# FASCICULE DE BREVET EUROPEEN

(11) **EP 2 996 623 B1**
(45) Date de publication et mention de la délivrance du brevet: **11.10.2017**
(21) Numéro de dépôt: 14723839.8
(22) Date de dépôt: 10.04.2014
(51) Int. Cl.: A61B 90/90

(54) **TRAÇABILITÉ DES INSTRUMENTS CHIRURGICAUX DANS UNE ENCEINTE HOSPITALIÈRE**
VERFOLGBARKEIT VON CHIRURGISCHEN INSTRUMENTEN IN EINEM KRANKENHAUVERBUND
TRACEABILITY OF SURGICAL INSTRUMENTS IN A HOSPITAL COMPOUND

(30) Priorité: 10.04.2013 FR 1353240
(43) Date de publication de la demande: 23.03.2016
(73) Titulaire: ANALYTIC - Traçabilité Hospitalière, 59721 Denain Cedex (FR)
(72) Inventeur: BRONINX, Geoffrey, 59293 Neuville-sur-Escaut (FR)
(74) Mandataire: Cabinet Rifflart Vandenbossche
(86) Numéro de dépôt international: PCT/FR2014/050863
(87) Numéro de publication internationale: WO 2014/167252

(56) Documents cités:
- EP-A1- 2 581 863
- DE-U1-202011 050 001
- US-A1- 2004 186 683
- US-A1- 2008 185 430
- US-A1- 2009 272 806
- US-A1- 2012 259 582
- US-B1- 6 707 381

## Description

### Domaine technique et Art antérieur

L'objet de la présente invention concerne le domaine de la sécurité sanitaire, et porte plus précisément sur la traçabilité et le suivi des instruments chirurgicaux dans un environnement médical tel qu'une enceinte hospitalière.

L'objet de la présente invention a pour objectif de répondre aux différentes normes sécuritaires et sanitaires qui sont désormais en vigueur en matière de traçabilité et de suivi des instruments chirurgicaux (non marqués) dans les enceintes hospitalières telles que les hôpitaux ou les cliniques.

La présente invention trouve naturellement une application particulièrement avantageuse dans les hôpitaux ou les cliniques en permettant la traçabilité et le suivi des instruments chirurgicaux ainsi que la recomposition d'un kit chirurgical composé d'une pluralité d'instruments chirurgicaux.

D'autres applications avantageuses de la présente invention peuvent également être envisagées par exemple chez un vétérinaire ou chez un dentiste.

Par instrument chirurgical au sens de la présente invention, il faut comprendre ici dans toute la description qui suit tout type d'instrument ou de dispositif médical destiné à être utilisé par un praticien tel qu'un médecin ou un chirurgien lors d'une intervention médicale ou chirurgicale. Il peut s'agir à titre d'exemples non limitatifs d'un instrument du type notamment ancillaire, lame de bistouris, pince (hémostatique, cystoscopique, à champs, intestinale, etc.), vis, instrument à ligatures, écarteur, ou encore sonde.

Les hôpitaux et les cliniques sont de plus en plus soumis à des réglementations régissant la sécurité sanitaire des patients.

Parmi ces réglementations, l'instruction DGS/RI3/2011/449 du 1^{er} décembre 2011 préconise la traçabilité unitaire des dispositifs médicaux réutilisables pour la lutte contre les infections du type Creutzfeldt-Jakob et ses variantes.

On comprend que les instruments chirurgicaux au sens de la présente invention font partie de ces dispositifs médicaux réutilisables.

Plus particulièrement, cette instruction assure la continuité de la circulaire « 138 » de 2001 qui requiert la traçabilité des cinq derniers patients pour un instrument chirurgical.

Le décret n° 2006-1497 précise, quant à lui, les contours de la traçabilité des dispositifs médicaux en définissant "*les règles particulières de la matériovigilance exercée sur certains dispositifs*". Le texte de ce décret précise que la matériovigilance comporte des règles de traçabilité depuis la réception des dispositifs médicaux dans l'enceinte hospitalière jusqu'à son utilisation sur le patient.

Aujourd'hui, cette traçabilité des instruments chirurgicaux est principalement assurée selon deux approches.

Selon une première approche, l'opérateur responsable de la traçabilité (appelé généralement le pharmacien) doit identifier visuellement chaque instrument chirurgical à partir de ses connaissances propres et/ou à partir de photographies de référence.

Il entre ensuite manuellement la référence de l'instrument dans un système informatique permettant le suivi des instruments chirurgicaux dans l'enceinte hospitalière ; suite à ce référencement, il positionne chaque instrument dans un kit chirurgical en vue d'une future opération au bloc.

Cette approche nécessite un travail long et fastidieux.

Par ailleurs, une telle approche ne répond pas aux nouvelles exigences sanitaires mentionnées ci-dessus : le risque d'erreur humaine lors d'une identification visuelle des instruments n'est pas négligeable.

Une deuxième approche consiste à utiliser un lecteur optique du type « *WhiteReader*® » pour lire un code 2D (par exemple un code du type « *DataMatrix* ») marqué ou gravé directement sur chaque instrument chirurgical. Alternativement, une identification par étiquettes RFID collées sur l'instrument chirurgical peut également être utilisée.

Ensuite, une fois cette lecture réalisée, le système informatique identifie automatiquement l'instrument en fonction de l'information contenue dans le code ou sur l'étiquette ; la référence de l'instrument ainsi que les informations correspondantes sont ensuite intégrée automatiquement dans le système.

La Demanderesse soumet qu'une telle approche présente des limites, notamment du fait qu'elle s'adresse exclusivement aux instruments chirurgicaux dits « marqués », que ce soit par gravure/marquage d'un code 2D ou par collage d'une étiquette RFID.

Or, les instruments chirurgicaux présentent diverses formes et dimensions qui peuvent empêcher un tel marquage. La Demanderesse soumet de plus qu'il est bien souvent très difficile de lire correctement ces codes 2D notamment lorsque les instruments sont utilisés lors d'une intervention chirurgicale (le code est recouvert de sang).

Les codes se dégradent également au cours du temps ; ceci est dû par exemple à l'usure induite par les cycles de stérilisations.

Par ailleurs, ces instruments chirurgicaux présentent le plus souvent des surfaces techniques sur lesquelles il est impossible de graver/marquer un code ou de coller une étiquette RFID, à défaut d'en altérer les fonctions.

On observera de plus que certains instruments chirurgicaux tels que les ancillaires sont spécifiquement conçus pour des interventions dédiées : ces instruments sont généralement prêtés par des industriels aux chirurgiens qui par exemple posent les implants achetés aux industriels. Dans pareils cas, ces ancillaires restent bien souvent la propriété des industriels, et ne peuvent donc pas être marqués de façon indélébile par les hôpitaux ou les cliniques.

Le document US 2012/0259582 concerne les instruments chirurgicaux non-marqués et propose une approche pour remédier, au moins partiellement, aux différents inconvénients ci-dessus.

Plus particulièrement, dans ce document, on propose l'utilisation d'une plateforme intégrant une pluralité de capteurs de poids aptes à établir une distribution « géographique » de la répartition du poids d'un instrument chirurgical posé sur la plateforme.

Cette plateforme est combinée à un système informatique comprenant une base de données préétablie dans laquelle sont stockées les informations relatives à la répartition du poids d'une pluralité d'instruments chirurgicaux prédéterminés. Il est ainsi possible, en connaissant cette distribution, de déterminer l'instrument qui est posé sur la plateforme.

La Demanderesse soumet toutefois que cette approche, bien qu'intéressante, n'est adaptée qu'aux instruments « plats » tels que ceux représentés aux figures 6 à 12 de ce document US 2012/0259582.

En effet, selon cette approche, la détermination de l'instrument chirurgical dépend directement de la façon dont on pose l'instrument sur la plateforme.

Ainsi, la solution proposée dans ce document ne convient aux instruments chirurgicaux possédant une forme en trois dimensions, présentant plusieurs faces d'appui et pouvant ainsi être posés selon plusieurs faces.

Dans le document US 2004/0186683, il est proposé d'utiliser conjointement une caméra et des capteurs de poids. Toutefois, l'utilisation de la caméra vise essentiellement à identifier en temps réel qu'un instrument vient d'être pris par un chirurgien pendant une opération.

En effet, selon cette approche, il est requis de positionner chaque type d'instrument selon un emplacement prédéfini. Lorsqu'on retire un instrument de son emplacement, ceci est détecté par la caméra.

Avec une telle approche, il faut un grand nombre d'emplacements car un kit ancillaire contient généralement une grande variété d'instruments ; la solution technique préconisée dans ce document US 2004/0186683 n'est pas adaptée à la réalité du terrain.

Par ailleurs, cette solution est intéressante pour le suivi des instruments pendant une intervention chirurgicale ; cependant, celle-ci n'est pas prévue pour recomposer un kit chirurgical après opération. Le document DE202011050001U U1 décrit un procédé et un appareil d'identification d'instruments chirurgicaux reposant sur une mesure de poids ainsi que sur une reconnaissance de forme utilisant une caméra. Une base de données permet de faire la correspondance entre les mesures et l'instrument. L'état de la technique ne propose pas de solution entièrement satisfaisante permettant de garantir à tout moment et dans toutes circonstances la traçabilité des instruments chirurgicaux dans une enceinte hospitalière.

### Objet et résumé de la présente invention

L'objet de la présente invention vise à améliorer la situation actuelle décrite ci-dessus.

L'objet de la présente invention vise à remédier aux différents inconvénients mentionnés ci-dessus en proposant un procédé de traçabilité des instruments chirurgicaux dans une enceinte hospitalière, un tel procédé étant prévu par exemple pour la recomposition d'un kit chirurgical composé d'un ou plusieurs instruments chirurgicaux.

Selon la présente invention, le procédé est mis en oeuvre par des moyens informatiques et comporte notamment une phase d'acquisition et une phase de traitement.

Plus particulièrement, la phase d'acquisition selon la présente invention comporte les étapes suivantes :
- un positionnement d'un instrument chirurgical sur une plateforme de support ;
- une capture, à l'aide d'une caméra numérique positionnée au-dessus de la plateforme de support, d'au moins une image numérique comprenant une vue de dessus d'au moins une portion de l'instrument chirurgical ; et
- un traitement d'images pour détecter dans ladite au moins une image numérique au moins une caractéristique visuelle de l'instrument chirurgical et pour déterminer au moins une donnée visuelle associée à cette caractéristique visuelle.

La phase d'acquisition comporte en outre une mesure du poids de l'instrument chirurgical, à l'aide d'une balance numérique telle qu'une balance de précision, afin de recueillir une donnée de poids contenant l'information relative au poids mesuré.

Suite à cette phase d'acquisition, le procédé comporte une phase de traitement comportant une identification de l'instrument chirurgical.

Plus particulièrement, cette identification consiste notamment à comparer la donnée de poids et la donnée visuelle avec une base de données prédéterminée établissant un lien entre au moins un instrument chirurgical et les informations relatives au poids et à une ou plusieurs caractéristiques visuelles dudit au moins un instrument chirurgical.

Cette identification consiste également à déterminer, en fonction de cette comparaison, une donnée d'identification contenant une information relative à l'identité de l'instrument chirurgical posé sur la plateforme de support.

De préférence, cette comparaison consiste à comparer chaque donnée de poids avec l'ensemble des données de poids contenues dans la base de données, et à calculer une « distance » entre la donnée de poids mesurée et chacune de ces données de poids de la base de données ; de la même façon, chaque donnée visuelle est comparée avec l'ensemble des données visuelles contenues dans la base de données pour calculer une « distance » entre la donnée visuelle mesurée et chacune de ces données.

Ces « distances » calculées peuvent être éventuellement pondérées, par exemple si on considère que la ou les caractéristiques visuelles mesurées sont plus discriminantes que le poids mesuré.

L'ensemble de ces distances calculées (et éventuellement pondérées) permett de déterminer une liste de candidats rangés selon un indice de confiance qui est fonction de ces « distances ».

La détermination de la donnée d'identification consiste ensuite à sélectionner le candidat présentant la plus petite « distance » ; c'est-à-dire le candidat qui présente le meilleur indice de confiance pour l'identification de l'instrument.

Le calcul de cette distance peut se faire par exemple en calculant la valeur absolue d'une différence, ou encore en calculant le ratio des deux valeurs. L'homme du métier comprendra ici que d'autres formules peuvent également être retenues pour ce calcul de « distance ».

Ainsi, cette succession d'étapes techniques, caractéristique de la présente invention, permet d'identifier automatiquement les instruments chirurgicaux pour en assurer un suivi et la traçabilité, ceci par exemple pour recomposer correctement un kit chirurgical.

Cette identification ne requiert aucun marquage ni aucune altération fonctionnelle des instruments chirurgicaux, et permet astucieusement de remédier aux inconvénients identifiés dans l'état de la technique décrit ci-dessus.

L'exploitation des techniques de traitement d'images permet ici de déterminer de façon précise des caractéristiques intrinsèques de l'instrument chirurgical telles par exemple sa forme, ses dimensions, ses contours ou encore sa couleur.

La mesure du poids permet ensuite de déterminer une autre caractéristique intrinsèque de l'instrument chirurgical.

L'association de ces caractéristiques intrinsèques, propres à l'instrument, permet d'identifier l'instrument avec un bon niveau de précision.

Cette identification implique que la base de données comporte l'information relative aux caractéristiques visuelles et au poids de chaque instrument chirurgical.

Le procédé selon l'invention prévoit de préférence un apprentissage pour la constitution de cette base de données servant de référentiel à l'identification des instruments.

Au cours de cet apprentissage, l'opérateur peut alors saisir le poids de tous les instruments chirurgicaux dont il dispose, et saisir des clichés de ces instruments pour lesquels il enregistre et valide des caractéristiques visuelles. Ainsi, le procédé selon l'invention comprend une étape préalable d'apprentissage au cours de laquelle la base de données est construite en associant pour chaque instrument chirurgical déterminé les informations dites intrinsèques relatives au poids et aux caractéristiques visuelles dudit instrument chirurgical.

Lors de cet apprentissage, chaque instrument est référencé avec les caractéristiques intrinsèques qui lui sont propres : poids, forme, couleur, contour, impédance, etc.

Avantageusement, la base de données peut également être alimentée lorsqu'un nouvel instrument est disponible (par exemple lors d'une une mise à jour), ou suite à une validation de l'identification.

On peut prévoir dans une variante que la base de donnée est une base de données centralisée connectée à un serveur distant ; dans ce cas, l'étape d'identification comprend l'interrogation à distance de cette dite base de donnée.

Le fait de déporter l'administration et la gestion de cette base de données permet d'avoir une base de données à jour. L'alimentation de cette base de données est centralisée, ce qui limite la responsabilité de l'utilisateur et réduit le risque sanitaire lié à une mauvaise identification à cause d'une base de données qui n'est pas à jour.

Avantageusement, la plateforme de support sur laquelle est posé l'instrument chirurgical est diffusante ; c'est-à-dire qu'elle laisse passer la lumière ou plus particulièrement permet la diffusion de la lumière.

De préférence, la phase d'acquisition comprend un rétro-éclairage de l'instrument chirurgical en émettant une lumière de rétro-éclairage, à travers la plateforme de support en direction de la caméra numérique.

Ce rétro-éclairage permet avantageusement de dégager la silhouette de l'instrument chirurgical, et d'éviter notamment les zones d'ombre qui pourraient altérer la forme détectée de l'instrument et introduire de la sorte une erreur dans l'identification.

Un tel rétro-éclairage est avantageux par exemple lors de la mesure de certaines caractéristiques comme la forme ou les contours. On comprend que la mesure de la caractéristique visuelle relative à la couleur de l'instrument ne nécessite pas ce rétro-éclairage.

Cette lumière de rétro-éclairage est de préférence émise à la verticale ; c'est-à-dire dans une direction sensiblement perpendiculaire au plan formé par la plateforme. Une telle orientation évite que la forme de l'instrument posé sur la plateforme ne soit déformée. Il faut également comprendre que cette lumière de rétro-éclairage est émise en direction de la caméra numérique qui est positionnée au-dessus de la plateforme.

Avantageusement, la lumière de rétro-éclairage présente une longueur d'onde comprise entre de l'ordre de 400 et 500 nanomètres ; de préférence, cette longueur d'onde est sensiblement égale à 470 nanomètres.

Avantageusement, la balance est configurée pour couvrir une plage de poids compris entre de 0 et 10 kilogrammes, de préférence avec une précision comprise entre 0 et 1 gramme.

De préférence, la phase d'acquisition comprend en outre une polarisation de la lumière de rétro-éclairage par un film diffuseur recouvrant la plateforme de support pour homogénéiser la diffusion de la lumière de rétro-éclairage passant à travers ladite plateforme.

Cette polarisation permet ainsi d'homogénéiser l'éclairage dans le champ de vision de la caméra numérique.

Plus particulièrement, cette lumière de rétro-éclairage est diffusée dans une unique direction, qui de préférence est celle de la caméra. On parle de film diffuseur unidirectionnel,

Ceci évite que l'opérateur soit ébloui.

De préférence, le film diffuseur unidirectionnel est configuré pour permettre la diffusion de la lumière de rétro-éclairage à travers la plateforme dans une direction perpendiculaire au plan défini par la plateforme de support.

Dans une variante, la phase d'acquisition comprend en outre une mesure d'une autre grandeur physique caractéristique de l'instrument chirurgical.

Dans cette variante, l'identification comprend une vérification de la donnée d'identification en fonction de cette grandeur mesurée ; ici, la base de données comprend pour chaque instrument chirurgical une information prédéterminée relative à cette grandeur physique.

Avantageusement, cette grandeur physique correspond à une caractéristique électrique de l'instrument telle que son impédance.

Dans cette variante, on comprend donc que l'impédance permet elle aussi grâce à la base de données de caractériser et d'identifier l'instrument chirurgical.

L'homme du métier comprendra ici que d'autres grandeurs peuvent être mesurées lors de cette phase d'acquisition.

Ces différentes variantes de réalisation décrites ci-dessus permettent de faire une discrimination des caractéristiques intrinsèques des instruments pour identifier avec précision l'instrument chirurgical posé sur la plateforme. Cette identification est ensuite soumise à une validation manuelle par l'utilisateur.

Cette validation manuelle permet d'enrichir la précision de la base de données au fur et à mesure de son utilisation.

Il est également prévu une combinaison des différentes variantes d'acquisition décrites ci-dessus, ainsi que des moyens de vérification qui calculent un indice de confiance des résultats d'identification permettant à l'utilisateur d'avoir une idée sur la fiabilité du résultat.

Ces moyens peuvent également être configurés pour pondérer les résultats de chaque identification en fonction de chaque variable mesurée pour obtenir une identification finale précise. Par exemple, lorsque les résultats de l'identification par les caractéristiques visuelles et la valeur du poids sont les mêmes, alors l'identification est validée.

Avantageusement, la phase de traitement peut comporter une étape préalable d'agrégation de la donnée de poids et de la ou des données visuelles.

Permettre la préparation des instruments chirurgicaux dédiés en vue d'une intervention chirurgicale est un des autres objectifs de la présente invention.

Avant une telle intervention, les différents instruments chirurgicaux requis pour l'intervention sont apportés au bloc dans un kit après stérilisation.

Il est nécessaire d'avoir une connaissance précise des différents instruments qui sont dans le kit ainsi qu'un historique de leurs précédentes utilisations.

Il est également impératif que chaque instrument soit correctement positionné dans le kit à l'emplacement qui lui est dédié pour en faciliter les manipulations pendant l'intervention.

Ainsi, selon l'invention, la base de données comporte pour chaque instrument chirurgical, une donnée de localisation contenant une information relative à un emplacement prédéterminé de l'instrument chirurgical dans le kit chirurgical.

Selon la présente invention, le procédé comporte alors une phase de recomposition comprenant une étape de localisation au cours de laquelle l'emplacement de l'instrument chirurgical dans le kit chirurgical est déterminé en fonction de cette donnée de localisation.

Avantageusement, le procédé selon l'invention comporte en outre une étape de suivi qui consiste à assigner à la donnée d'identification une information relative à l'utilisation de l'instrument. Ceci permet par exemple de signaler un instrument lorsque celui-ci a été utilisé lors d'une intervention chirurgicale, ou de signaler un instrument lorsque celui-ci a été stérilisé avant l'intervention chirurgicale.

Ainsi, il est possible d'établir un historique des évènements attachés à un instrument chirurgical par exemple en le signalant comme « utilisé » ou « non utilisé », et/ou en le signalant comme « stérilisé » ou « non stérilisé ». Selon l'invention, la phase de recomposition comporte en outre une étape d'affichage qui consiste à afficher sur un écran numérique de type tablette numérique une cartographie numérique du kit chirurgical ainsi que les instruments chirurgicaux associés selon leur emplacement prédéterminé.

L'affichage d'une telle cartographie est avantageux dans la mesure où il permet de visualiser aisément le kit et les instruments qui y sont associés. L'affichage de la localisation sert également à avoir un « état » de la recomposition, en listant les instruments qui ont déjà été placés dans le kit et ceux qui ne le sont pas encore, pour visualiser à termes les éventuels instruments manquants (qui seraient oubliés au bloc dans le laveur, ou encore seraient jetés par mégarde à la poubelle, etc.).

De préférence, l'écran numérique est tactile, et il est possible de sélectionner manuellement sur l'écran l'instrument désiré, par exemple lorsque le praticien qui opère utilise l'instrument.

Corrélativement, l'objet de la présente description porte sur un programme d'ordinateur qui comporte des instructions adaptées pour l'exécution des étapes du procédé tel que décrit ci-dessus, ceci notamment lorsque ledit programme d'ordinateur est exécuté par au moins un processeur.

Un tel programme d'ordinateur peut utiliser n'importe quel langage de programmation, et être sous la forme d'un code source, d'un code objet, ou d'un code intermédiaire entre un code source et un code objet, tel que dans une forme partiellement compilée, ou dans n'importe quelle autre forme souhaitable.

De même, l'objet de la présente description porte sur un support d'enregistrement lisible par un ordinateur sur lequel est enregistré un programme d'ordinateur comprenant des instructions pour l'exécution des étapes du procédé tel que décrit ci-dessus.

D'une part, le support d'enregistrement peut être n'importe quel entité ou dispositif capable de stocker le programme. Par exemple, le support peut comporter un moyen de stockage, tel qu'une mémoire ROM, par exemple un CD-ROM ou une mémoire ROM de type circuit microélectronique, ou encore un moyen d'enregistrement magnétique, par exemple une disquette de type « *floppy disc* » ou un disque dur (type stockage de masse : disque dur ou clé USB).

D'autre part, ce support d'enregistrement peut également être un support transmissible tel qu'un signal électrique ou optique, un tel signal pouvant être acheminé via un câble électrique ou optique, par radio classique ou hertzienne ou par faisceau laser autodirigé ou par d'autres moyens. Le programme d'ordinateur peut être en particulier téléchargé sur un réseau de type Internet.

Alternativement, le support d'enregistrement peut être un circuit intégré dans lequel le programme d'ordinateur est incorporé, le circuit intégré étant adapté pour exécuter ou pour être utilisé dans l'exécution du procédé en question.

L'objet de la présente invention porte également sur un dispositif informatique de traçabilité des instruments chirurgicaux dans une enceinte hospitalière pour la recomposition d'un kit chirurgical composé d'au moins un instrument chirurgical.

Le dispositif selon la présente invention comporte des moyens informatiques configurés pour la mise en oeuvre du procédé tel que décrit ci-dessus.

Plus précisément, le dispositif comporte avantageusement :
- un module d'acquisition comprenant :
   une plateforme de support destinée à recevoir un unique instrument chirurgical,
   une caméra numérique, positionnée au-dessus de la plateforme de support, configurée pour saisir au moins une image numérique comprenant une vue de dessus d'au moins une portion de l'instrument chirurgical,
   un circuit électronique de traitement d'images configuré pour détecter dans ladite au moins une image numérique au moins une caractéristique visuelle dudit instrument chirurgical et pour déterminer au moins une donnée visuelle associée à cette caractéristique,
   une balance numérique configurée pour mesurer le poids de l'instrument chirurgical et pour déterminer une donnée de poids contenant l'information relative au poids mesuré ;
      - un module de traitement comprenant :
         un circuit électronique d'identification configuré d'une part pour comparer la donnée de poids et la donnée visuelle avec une base de données prédéterminée établissant un lien entre au moins un instrument chirurgical et les informations relatives au poids et à la caractéristique visuelle dudit au moins un instrument chirurgical, et d'autre part pour déterminer en fonction de ladite comparaison et de la base de données au moins une donnée d'identification contenant une information relative à l'identité dudit instrument chirurgical. La base de données comporte, pour chaque instrument chirurgical, une donnée de localisation contenant une information relative à un emplacement prédéterminé dudit instrument chirurgical dans un kit chirurgical. Le dispositif comporte un circuit électronique de traçabilité configuré pour déterminer l'emplacement dudit instrument chirurgical dans ledit kit chirurgical en fonction de ladite donnée de localisation et comporte des moyens d'affichage tels qu'un écran numérique configurés pour afficher une cartographie numérique du kit chirurgical ainsi que les instruments chirurgicaux associés selon leur emplacement.

Ainsi, la présente invention, grâce à la combinaison de ses caractéristiques techniques fonctionnelles et structurelles, permet le suivi et la traçabilité des instruments chirurgicaux dans une enceinte hospitalière en garantissant notamment l'exactitude et l'exhaustivité des instruments qui sont positionnés dans un kit.

### Brève description des figures annexées

D'autres caractéristiques et avantages de la présente invention ressortiront de la description ci-dessous, en référence aux figures 1 à 3 annexées qui en illustrent un exemple de réalisation dépourvu de tout caractère limitatif et sur lesquelles :
- la figure 1 représente de façon schématique un dispositif de traçabilité des instruments chirurgicaux selon un exemple de réalisation avantageux de la présente invention ;
- la figure 2 représente de façon schématique un exemple non limitatif d'un écran numérique affichant la cartographie virtuelle d'un kit chirurgical et des instruments médicaux associés ; et
- la figure 3 consiste en un organigramme représentatif des différentes étapes mises en oeuvre selon un exemple de réalisation du procédé de l'invention.

### Description détaillée d'un mode de réalisation de l'invention

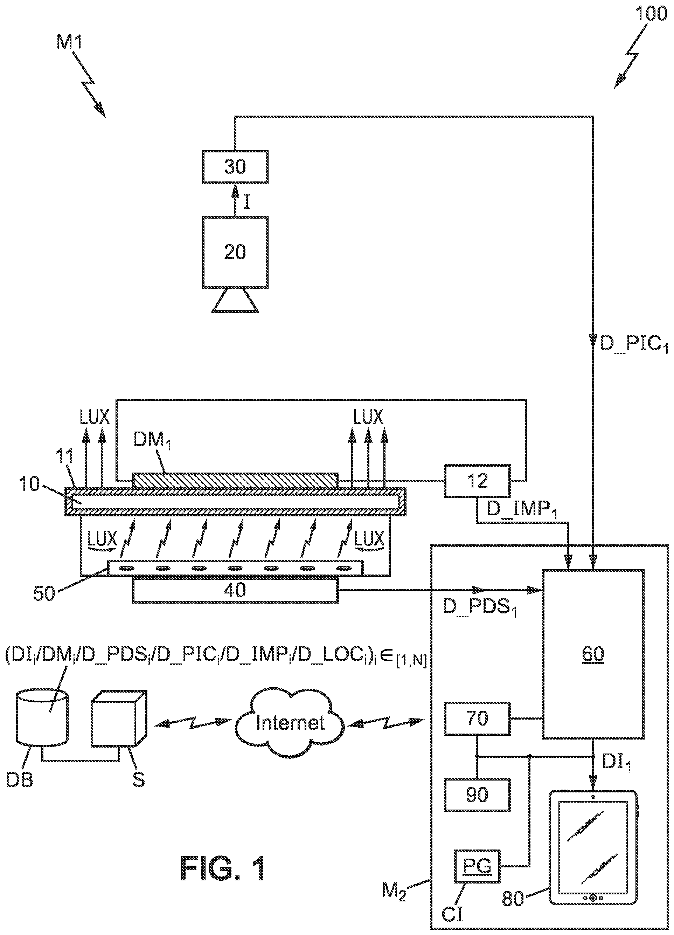

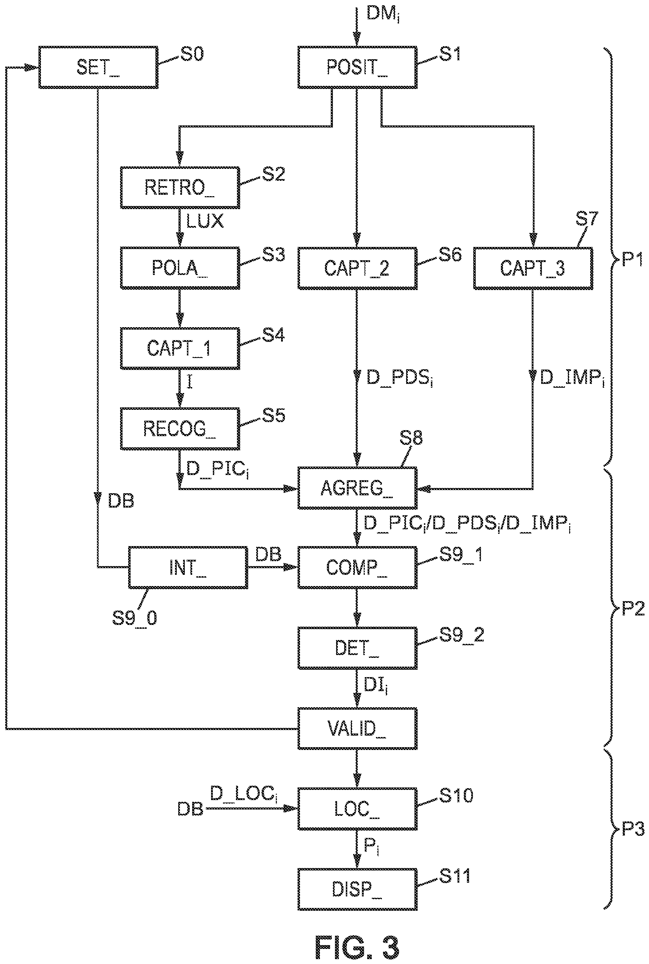

Un procédé pour la traçabilité des instruments chirurgicaux dans une enceinte hospitalière ainsi que le dispositif associé vont maintenant être décrits dans ce qui suit en faisant référence conjointement aux figures 1 à 3.

Permettre la traçabilité des instruments chirurgicaux remédiant aux différents inconvénients identifiés ci-dessus dans l'état de la technique est un des objectifs de la présente invention.

A cet effet, la présente invention met à disposition de l'opérateur en charge de recomposer un kit chirurgical KT un outil simple et facile d'utilisation qui consiste en un dispositif de traçabilité 100 ; ce dispositif 100 permet une identification précise des instruments chirurgicaux DMᵢ (où i étant un entier positif) en exploitant des informations physiques qui sont caractéristiques des instruments.

La variable i est ici égale à 3 pour simplifier l'exemple décrit dans les figures.

Ainsi, on dispose donc dans cet exemple des instruments chirurgicaux DM₁, DM₂, et DM₃.

Dans cet exemple et comme illustré en figure 1, l'opérateur positionne lors d'une étape S1 l'instrument chirurgical qu'il doit identifier, ici DM₁, sur une plateforme de support 10. De préférence, cette plateforme 10 présente une forme incurvée (non représentée ici) pour éviter que le dispositif ne tombe par terre et s'abîme.

Une fois positionné sur cette plateforme 10, l'opérateur actionne le dispositif 100.

Plus particulièrement, le dispositif 100 comporte un module d'acquisition M1 configuré pour acquérir lors d'une phase d'acquisition P1 plusieurs signaux, de nature électrique, contenant chacun une information relative à une caractéristique intrinsèque de l'instrument chirurgical posé sur la plateforme de support 10 ; c'est-à-dire une caractéristique propre à l'instrument qui permet de caractériser ledit instrument.

Les différentes informations contenues dans chacun de ces signaux électriques seront ensuite exploitées lors d'une deuxième phase dite de traitement P1.

Un des premiers aspects de la présente invention consiste à exploiter les caractéristiques visuelles de l'instrument DM₁ posé sur la plateforme 10 ; il peut s'agir par exemple de la forme de l'instrument, son contour, un rayon de courbure spécifique, chacun de ces éléments picturaux étant susceptible de caractériser l'instrument DM₁.

Ainsi, dans l'exemple décrit ici, le module d'acquisition M1 comprend une caméra numérique 20 et un circuit électronique de traitement d'images 30, tel qu'un processeur de traitement d'images, configuré pour le traitement d'images.

Selon la présente invention, il peut s'agir d'une caméra 2D ou d'une caméra 3D (par exemple une caméra du type « *Time of Flight* » ou un scanner à lumière structurée).

Dans l'exemple décrit ici, la caméra 20 permet donc d'acquérir une image I de l'instrument DM₁, lors d'une étape S4.

Préalablement à cette étape d'acquisition S4, le module d'acquisition M1 prévoit l'émission d'une source lumineuse LUX, dite de rétro-éclairage, lors d'une étape S2, par une pluralité de LEDs 50 ; ces LEDs 50 sont positionnées sous la plateforme 10 et émettent une lumière de rétro-éclairage LUX à travers la plateforme 10.

On comprend ici que, dans cet exemple, la plateforme 10 est diffusante ; elle permet ainsi la diffusion de la lumière de rétro-éclairage.

Dans l'exemple décrit ici, l'émission de cette lumière LUX se fait à la verticale en direction de la caméra 20.

Ce rétro-éclairage S2 permet ainsi d'éviter les zones d'ombre dans le champ de vision de la caméra 20.

Dans l'exemple décrit ici, la longueur d'onde de cette lumière de rétro-éclairage LUX est sensiblement égale à 470 nanomètres. Cette longueur d'onde présente l'avantage d'assurer une bonne stabilité de la luminosité ambiante lors de la capture d'image S4 ; elle permet en outre de dégager correctement les contrastes dans l'environnement de travail.

Afin d'homogénéiser cette lumière LUX dans le champ de vision de la caméra 20 pour avoir une image nette, la plateforme 10 est recouverte d'un film diffuseur 11 qui permet une polarisation S3 de la lumière LUX dans une même direction.

On parle de film diffuseur unidirectionnel.

De préférence, ce film diffuseur unidirectionnel 11 est configuré pour diffuser la lumière sensiblement dans la direction de la caméra 20 ; c'est-à-dire ici perpendiculairement à la plateforme 10.

Cette polarisation de la lumière évite notamment que l'opérateur ne soit ébloui.

Grâce à ce rétro-éclairage S2 et cette polarisation S3, l'image I saisie par la caméra 20 est nette et met en évidence la forme de l'instrument DM₁ en évitant les zones d'ombres.

Afin de reconnaître dans cette image numérique I une ou plusieurs caractéristiques visuelles de l'instrument chirurgical DM₁, le circuit électronique de traitement d'images 30 est configuré pour effectuer un traitement d'images S5 permettant de détecter et d'extraire des caractéristiques visuelles dans l'image I ; le circuit électronique 20 est ainsi configuré pour exploiter des techniques de traitement d'images du type par exemple les techniques d'extraction de l'arrière-plan, et/ou pour déterminer des coefficients de description de formes du type par exemple Zernike ou Hue, ou de contours.

Alternativement et/ou de façon complémentaire, elle peut se faire en calculant les dimensions.

Cette reconnaissance S5 peut également se faire par reconnaissance de caractéristiques « locales » du type par exemple FAST ou MSER.

Dans l'exemple décrit, le processeur 30 est également configuré pour exploiter les informations relatives aux éléments colorimétriques de l'image I acquise : par exemple en exploitant un histogramme de couleurs ou les moments colorimétriques.

En posant l'instrument DM₁ sur la plateforme 10, il est ainsi possible de déterminer au moins une donnée visuelle D_PIC₁ caractéristique de l'instrument DM₁.

Le module d'acquisition M1 permet d'exploiter d'autres caractéristiques physiques caractéristiques de l'instrument, par exemple son poids ou son impédance.

Dans l'exemple décrit ici, et comme illustré en figure 1, le module d'acquisition M1 comprend ainsi une balance de précision 40, intégrée dans la plateforme.

Ainsi, lorsque l'opérateur positionne l'instrument chirurgical DM₁ sur la plateforme 10, il obtient lors d'une étape S6 une donnée D_PDS₁ contenant l'information relative au poids de cet instrument DM₁.

Dans l'exemple décrit ici, le module d'acquisition M1 peut en outre exploiter une autre caractéristique de l'instrument chirurgical DM₁, par exemple son impédance.

Dans ce mode, le module M1 comporte ainsi un moyen de mesure électrique 12 configuré pour mesurer lors d'une étape S7 un signal électrique représentatif de l'impédance de l'instrument chirurgical. Par cette troisième mesure, on connaît donc une donnée d'impédance D_IMP₁ relative à l'instrument chirurgical DM₁.

L'ensemble de ces données D_PIC₁, D_PDS₁, et D_IMP₁ issues de cette phase d'acquisition P1 sont toutes caractéristiques de l'instrument DM₁.

Ces données sont ensuite traitées et exploitées lors d'une phase P2 dite de traitement pour déterminer précisément l'identité de l'instrument posé sur la plateforme 10.

Tout d'abord, ces données D_PIC₁, D_PDS₁, et D_IMP₁ sont agrégées lors d'une étape S8 par un circuit électronique d'identification 60.

Une fois agrégées, il est prévu une identification S9 de l'instrument DM₁ posé sur la plateforme 10.

Cette identification S9 comprend une comparaison S9_1 par le circuit électronique 60 de la donnée de poids D_PDS₁, de la donnée visuelle D_PIC₁ et de la donnée d'impédance D_IMP₁ avec une base de données DB.

Cette base de données DB comprend les données d'une pluralité d'instruments DM₁, DM₂, et DM₃ et les informations prédéterminées relatives au poids et aux caractéristiques visuelles de chacun des instruments chirurgicaux.

Cette base DB contient également l'information relative à l'impédance de chacun de ces instruments chirurgicaux.

Dans l'exemple décrit ici, et comme illustré en figure 1, cette base de données DB est déportée ; elle est accessible via un serveur distant S.

Dans l'exemple décrit ici, et illustré en figure 1, la comparaison S9_1 nécessite au préalable une interrogation S9_0 par des moyens de communication 70 du serveur S qui est connecté à la base de données DB.

Comme évoqué précédemment, le fait de déporter l'administration et la gestion de cette base de données DB sur un serveur distant S permet d'avoir une base de données à jour.

L'alimentation de cette base de données DB est ainsi centralisée, ce qui limite la responsabilité de l'utilisateur et réduit le risque sanitaire lié à une mauvaise identification à cause d'une base de données qui n'est pas à jour.

Bien évidemment, l'homme du métier comprendra ici que la base de données DB peut également être administrée en local sur chaque dispositif 100.

Cette comparaison permet de faire un « *matching* » permettant au circuit électronique d'identification 60 de déterminer lors d'une étape S9_2 la donnée d'identification contenant l'information relative à l'identité de l'instrument chirurgical posé sur la plateforme.

Dans l'exemple décrit ici, le circuit électronique d'identification 60 compare donc les données D_PIC₁, D_PDS₁, et D_IMP₁ issues de la phase P1 avec les différentes données visuelles, de poids et d'impédance de chacun des instruments de la base de données DB.

Comme évoqué précédemment, lors de cette comparaison, on calcule pour chacune de ces données D_PIC₁, D_PDS₁, et D_IMP₁ issues de la phase P1 la distance avec les différentes données visuelles, de poids et d'impédance contenues dans la base de données DB.

Cette comparaison permet d'établir pour chacune des données D_PIC₁, D_PDS₁, et D_IMP₁ une liste de candidats possible en fonction des distances calculées (la plus petite) ; ce calcul de distance fournit en quelque sorte un indice de confiance de l'identification.

On calcule ensuite une distance dite globale, en fonction de coefficients de pondération prédéterminés.

Idéalement, cette pondération est égale à 1/n, n étant ici le nombre de données prise en considération (ici 3).

En sélectionnant la distance « globale » la plus petite, il est possible de déterminer la donnée DI₁ qui correspond dans la base DB à l'instrument DM₁; il s'agit bien évidemment ici d'un exemple particulier qui est purement illustratif et ne présente en aucun cas un caractère limitatif.

Le circuit électronique d'identification 60 est capable de déterminer automatiquement la donnée d'identification DIᵢ (soit DI₁, DI₂, ou DI₃) contenant l'information relative à la désignation de l'instrument DMᵢ (DMᵢ, DM₂, ou DM₃) posé sur la plateforme 10, en fonction des données recueillies lors de la phase d'acquisition P1.

Il est également possible de prévoir que le circuit 60 détermine une liste de candidats proposant la désignation de plusieurs instruments DMᵢ rangés par ordre de pertinence en fonction du calcul des distances et de leurs indices de confiance calculés.

Dans ce cas, c'est l'opérateur au final qui choisit dans cette liste l'instrument, en fonction notamment de son expérience.

Ceci permet un apprentissage est continu ; c'est-à-dire que l'opérateur peut également continuer à alimenter la base de données en validant l'identification ou compléter les informations qu'elle contient, par exemple pour y intégrer un nouvel instrument chirurgical ou enregistrer dans celle-ci une caractéristique non connue jusqu'à présent.

Dans l'exemple décrit ici, le circuit électronique d'identification 60 est également configuré pour corréler les différents résultats d'identification pour chaque signal mesuré, et éviter ainsi des erreurs d'identification ; une pondération de ces résultats est envisagée.

Ainsi, lorsque le résultat de l'identification n'est pas certain (indice de confiance inférieur à un seuil prédéterminé), par exemple lorsque la comparaison des données relatives aux caractéristiques visuelles et au poids avec la base de données aboutissent à une identification non-certaine, présentant un degré d'erreur non-négligeable, il est prévu que le système déclare l'instrument comme inconnu, laissant la validation à l'opérateur.

En fonction de l'identification que fera l'opérateur, le système peut mettre à jour la base de données et la pondération des distances des caractéristiques intrinsèques afin de reconnaître avec précision la fois suivante l'objet sur la plateforme.

Le circuit électronique d'identification 60 est ainsi configuré pour assurer une bonne identification de l'instrument chirurgical posé sur la plateforme, notamment en exploitant les données issues de la phase d'acquisition, en interrogeant une base de données à jour, et en procédant à une vérification.

Un des autres objectifs de la présente invention est d'assurer le suivi et la traçabilité des instruments chirurgicaux en vue de leur utilisation au bloc opératoire. Ces opérations font partie d'une phase de recomposition P3 permettant notamment la recomposition d'un kit chirurgical KT.

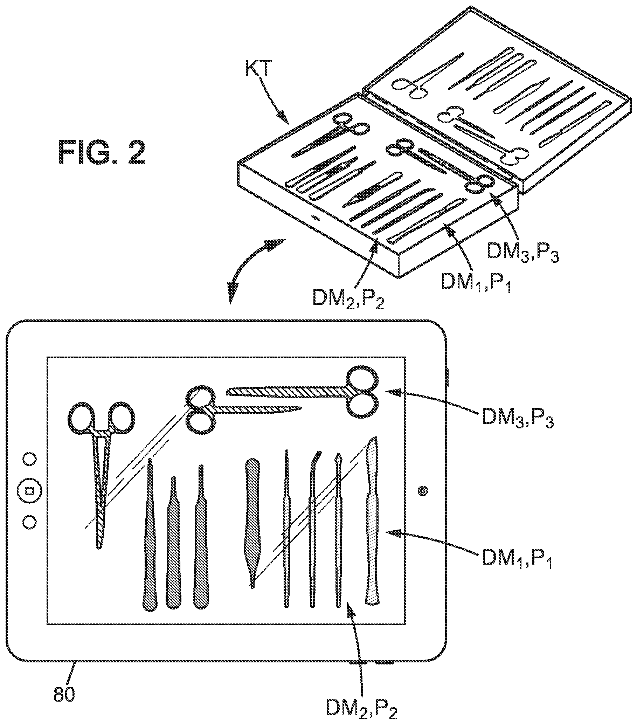

A cet effet, les instruments chirurgicaux sont généralement conditionnés dans un kit KT (représenté en figure 2).

Il est donc nécessaire d'avoir un kit KT correctement constitué, c'est-à-dire un kit KT dans lequel chaque instrument est positionné à l'emplacement qui lui est dédié.

Dans l'exemple décrit ici, et comme illustré ici en figures 1 et 2, la base de données DB comporte une donnée de localisation DL₁, DL₂, DL₃ fournissant un emplacement P₁, P₂, P₃ prédéterminé dans le kit KT pour chaque instrument chirurgical DM₁, DM₂, DM₃.

Dans cet exemple et comme illustré en figures 1 et 2, le dispositif 100 comporte un circuit électronique de traçabilité 90 qui est configuré pour, lors d'une étape de localisation S10, déterminer l'emplacement respectif P₁, P₂, P₃ de chaque instrument chirurgical DM₁, DM₂, DM₃ dans le kit KT en fonction de la donnée de localisation DL₁, DL₂, DL₃.

Dans l'exemple décrit ici, et comme illustré ici en figure 1, cette localisation S10 comprend un suivi au cours duquel est assignée à chaque donnée d'identification DI₁, DI₂, DI₃ une information relative à l'utilisation respectivement de chaque instrument DM₁, DM₂, DM₃ pour signaler les instruments DM₁, DM₂, DM₃ comme « utilisés » lors d'une intervention chirurgicale ou « stérilisés ».

Ainsi, chaque donnée d'identification DI₁, DI₂, DI₃ comporte l'information selon laquelle l'instrument DM₁, DM₂, DM₃ a été utilisé ou non (et/ou stérilisé ou non).

Dans l'exemple décrit ici, et comme illustré ici en figure 1, le dispositif de traçabilité 100 comporte des moyens d'affichage 80 tels qu'un écran numérique tactile de type tablette numérique configurés pour, lors d'une étape d'affichage S₁₁, afficher une cartographie numérique du kit chirurgical KT ainsi que les appareils chirurgicaux associés DM₁, DM₂, DM₃.

Comme illustré en figure 2, cet affichage permet de visualiser lors d'une intervention chirurgicale chaque instrument DM₁, DM₂, DM₃ dans le kit chirurgical KT, avec son emplacement précis et son historique (utilisé et/ou stérilisé).

A titre d'exemple, les instruments « grisés » sur la figure 2 sont des instruments utilisés, et les instruments « hachurés » sur la figure 2 sont des instruments stérilisés et non utilisés.

Les différents moyens décrits ci-dessus sont pilotés par un programme d'ordinateur PG spécifique qui comporte des instructions adaptées pour l'exécution des étapes du procédé décrit ci-dessus, ce programme PG est enregistré sur un support d'enregistrement CI.

Ainsi, la présente invention telle que décrite ci-dessus offre aux acteurs du monde hospitalier une solution innovante permettant de satisfaire aux nouvelles exigences sanitaires.

La présente invention propose ainsi un système de reconnaissance d'un instrument chirurgical (dispositif médical) sans marquage, à partir des caractéristiques physiques de celui-ci. Ce système vise essentiellement à combler le manque de dispositif de traçabilité automatique concernant les kits ancillaires, notamment lors de leur recomposition en stérilisation, les autres dispositifs médicaux disposant déjà d'une technologie mature à ce sujet.

La présente invention est ainsi particulièrement avantageuse pour être placée à deux endroits stratégiques : au bloc opératoire et à la stérilisation. A chacun de ces lieux correspond une utilisation spécifique : au bloc opératoire, la présente invention permet la pré-recomposition des kits KT. Plus précisément, à la fin d'une intervention, un opérateur se charge de recomposer le kit KT utilisé avant de l'envoyer en stérilisation. Il identifie chacun des instruments DMᵢ avant de les placer dans le kit KT. Ceci permet d'anticiper et de préparer l'étape de recomposition lors de la stérilisation (on facilite donc le travail de recomposition en stérilisation) ainsi que d'effectuer une traçabilité du kit en sortie de bloc. En stérilisation, la présente invention sert à la recomposition des kits KT. De manière similaire à l'utilisation en bloc, à la sortie de la stérilisation, un opérateur se charge de recomposer le kit stérilisé avant de l'envoyer au bloc opératoire. Chaque instrument DMᵢ est identifié un à un grâce à la présente invention. La recomposition du kit KT avant l'envoi au bloc est assurée, ce qui permet de limiter une re-stérilisation inutile. La traçabilité en sortie de stérilisation est également assurée.

L'avantage de la présente invention est d'avoir notamment une traçabilité complète, sans risque de stérilisation inutile et permettre à l'hôpital de la valoriser les instruments DMᵢ en volume et en valeur.

La présente invention peut être utilisée pour tout dispositif médical ayant besoin d'une identification et ne pouvant être marqué par un code ou par une étiquette.

Il devra être observé que cette description détaillée porte sur un exemple de réalisation particulier de la présente invention, mais qu'en aucun cas cette description ne revêt un quelconque caractère limitatif à l'objet de l'invention ; bien au contraire, elle a pour objectif d'ôter toute éventuelle imprécision ou toute mauvaise interprétation des revendications qui suivent.

## Revendications

1. Procédé de traçabilité des instruments chirurgicaux (DMᵢ, i étant un entier positif) dans une enceinte hospitalière, ledit procédé mis en oeuvre par des moyens informatiques comportant :
- une phase d'acquisition (P1) comportant :
un positionnement (S1) d'un instrument chirurgical (DMᵢ) sur une plateforme de support (10) ;
une capture (S4), à l'aide d'une caméra numérique (20) positionnée au-dessus de la plateforme de support (10), d'au moins une image numérique (I) comprenant au moins une portion de l'instrument chirurgical (DMᵢ);
un traitement d'images (S5) pour détecter dans ladite au moins une image numérique (I) au moins une caractéristique visuelle dudit instrument chirurgical (DMᵢ) et pour déterminer au moins une donnée visuelle (P_PICᵢ) associée à ladite caractéristique visuelle ;
une mesure (S6) du poids de l'instrument chirurgical (DMᵢ), à l'aide d'une balance numérique (40), afin de recueillir une donnée de poids (D_PDSᵢ) contenant l'information relative au poids mesuré ;
- une phase de traitement (P2) comportant une identification (S9) dudit instrument chirurgical (DMᵢ) consistant notamment à : comparer (S9_1) la donnée de poids (D_PDSᵢ) et la donnée visuelle (D_PICᵢ) avec une base de données (DB) établissant un lien entre au moins un instrument chirurgical (DMᵢ) et les informations relatives au poids et à la caractéristique visuelle dudit au moins un instrument chirurgical, et déterminer (S9_2), en fonction de ladite comparaison, au moins une donnée d'identification (DIᵢ) contenant une information relative à l'identité dudit instrument chirurgical (DMᵢ),
**caractérisé en ce que** la base de données (DB) comporte, pour chaque instrument chirurgical (DMᵢ), une donnée de localisation (DLᵢ) contenant une information relative à un emplacement (Pᵢ) prédéterminé dudit instrument chirurgical (DMᵢ) dans un kit chirurgical (KT), et **en ce qu'**il comporte une phase de recomposition (P3) dudit kit (KT) comprenant :
- une étape de localisation (10) au cours de laquelle l'emplacement (Pᵢ) dudit instrument chirurgical (DMᵢ) dans ledit kit chirurgical (KT) est déterminée en fonction de ladite donnée de localisation (DLᵢ), et
- une étape d'affichage (S11) consistant notamment à afficher sur un écran numérique (80) une cartographie numérique du kit chirurgical (KT) ainsi que les instruments chirurgicaux (DMᵢ) associés selon leur emplacement (Pᵢ).

2. Procédé selon la revendication 1, dans lequel la plateforme de support (10) sur laquelle est posé ledit instrument chirurgical (DMᵢ) est diffusante, et dans lequel la phase d'acquisition (P1) comprend un rétro-éclairage (S2) de l'instrument chirurgical (DMᵢ) en émettant une lumière de rétro-éclairage (LUX) à travers ladite plateforme (10) et en direction de ladite caméra numérique (20).

3. Procédé selon la revendication 2, dans lequel la lumière de rétro-éclairage (LUX) présente une longueur d'onde comprise entre de l'ordre de 400 et 500 nanomètres, de préférence sensiblement égale à 470 nanomètres.

4. Procédé selon la revendication 2 ou 3, dans lequel la phase d'acquisition (P1) comprend une polarisation (S3) de la lumière de rétro-éclairage (LUX) par un film diffuseur (11) unidirectionnel recouvrant ladite plateforme (10) pour homogénéiser la diffusion de la lumière de rétro-éclairage passant à travers ladite plateforme (10).

5. Procédé selon l'une quelconque des revendications précédentes, dans lequel la phase d'acquisition (P1) comprend une mesure (S7) de l'impédance dudit instrument chirurgical (DMᵢ), et dans lequel l'identification (S9) prend en considération ladite impédance pour déterminer la donnée d'identification (DIᵢ), ladite base de données (DB) comprenant pour chaque instrument chirurgical (DMᵢ) une information relative à une valeur d'impédance prédéterminée.

6. Procédé selon l'une quelconque des revendications précédentes, dans lequel la phase de traitement (P2) comporte une étape préalable d'agrégation (S8) de la donnée de poids (D_PDSᵢ) et de la donnée picturale (D_PICᵢ).

7. Procédé selon l'une quelconque des revendications précédentes, comprenant une étape préalable d'apprentissage (S0) au cours de laquelle la base de données (DB) est construite en associant pour chaque instrument chirurgical (DMᵢ) déterminé les informations relatives au poids et à au moins une caractéristique visuelle dudit instrument chirurgical.

8. Procédé selon l'une quelconque des revendications précédentes, dans lequel la base de données (DB) est une base de données centralisée connectée à un serveur distant (S) et dans lequel l'étape d'identification (S9) comprend l'interrogation (S9_0) à distance de cette dite base de donnée (DB).

9. Dispositif informatique de traçabilité (100) des instruments chirurgicaux (DMᵢ, i étant un entier positif) dans une enceinte hospitalière, ledit dispositif (100) comportant :
- un module d'acquisition (M1) comprenant :
une plateforme de support (10) destinée à recevoir un instrument chirurgical (DMᵢ, i étant un entier positif),
une caméra numérique (20), positionnée au-dessus de la plateforme de support (10), configurée pour saisir au moins une image numérique (I) comprenant au moins une portion de l'instrument chirurgical (DMᵢ),
un circuit électronique de traitement d'images (30) configuré pour détecter dans ladite au moins une image numérique (I) au moins une caractéristique visuelle dudit instrument chirurgical (DMᵢ) et pour déterminer au moins une données visuelle (P_PICᵢ) associée à ladite caractéristique visuelle,
une balance numérique (40) configurée pour mesurer le poids de l'instrument chirurgical (DMᵢ) et pour déterminer une donnée de poids (D_PDSᵢ) contenant l'information relative au poids mesuré ;
- un module de traitement (M2) comprenant :
un circuit électronique d'identification (60) configuré d'une part pour comparer la donnée de poids (D_PDSᵢ) et la donnée visuelle (D_PICᵢ) avec une base de données prédéterminée (DB) établissant un lien entre au moins un instrument chirurgical (DMᵢ) et les informations relatives au poids et à ladite caractéristique visuelle dudit au moins un instrument chirurgical, et d'autre part pour déterminer en fonction de ladite comparaison au moins une donnée d'identification (DIᵢ) contenant une information relative à la désignation dudit instrument chirurgical (DMᵢ),
**caractérisé en ce que** la base de données (DB) comporte, pour chaque instrument chirurgical (DMᵢ), une donnée de localisation (DLᵢ) contenant une information relative à un emplacement (Pᵢ) prédéterminé dudit instrument chirurgical (DMᵢ) dans un kit chirurgical (KT), et **en ce qu'**il comporte un circuit électronique de traçabilité (90) configuré pour déterminer l'emplacement (Pᵢ) dudit instrument chirurgical (DMᵢ) dans ledit kit chirurgical (KT) en fonction de ladite donnée de localisation (DLᵢ) et des moyens d'affichage (80) tels qu'un écran numérique configurés pour afficher une cartographie numérique du kit chirurgical (KT) ainsi que les instruments chirurgicaux (DMᵢ) associés selon leur emplacement (Pᵢ).

10. Dispositif de traçabilité (100) selon la revendication 9, comportant des moyens informatiques configurés pour la mise en oeuvre des étapes du procédé selon l'une quelconque des revendications 2 à 8.

## Patentansprüche

1. Verfahren zur Nachverfolgbarkeit von chirurgischen Instrumenten (DMᵢ, wobei i eine ganze positive Zahl ist) auf einem Krankenhausgelände, wobei das Verfahren durch Informatikmittel durchgeführt wird, die Folgendes umfassen:
- eine Erfassungsphase (P1), umfassend:
eine Positionierung (S1) eines chirurgischen Instruments (DMᵢ) auf einer Stützplattform (10);
eine Aufnahme (S4), mit Hilfe einer Digitalkamera (20), die über der Stützplattform (10) positioniert ist, von mindestens einem Digitalbild (I), das mindestens einen Abschnitt des chirurgischen Instruments (DMᵢ) aufweist;
eine Bildverarbeitung (S5), um in dem mindestens einen Digitalbild (I) mindestens eine visuelle Eigenschaft des chirurgischen Instruments (DMᵢ) nachzuweisen, und um mindestens ein visuelles Datum (P_PICᵢ) zu bestimmen, das mit der visuellen Eigenschaft assoziiert ist;
eine Messung (S6) des Gewichts des chirurgischen Instruments (DMᵢ) mit Hilfe einer digitalen Waage (40), um ein Gewichtsdatum (D_PDSᵢ) aufzunehmen, das die Information hinsichtlich des gemessenen Gewichts enthält;
- eine Verarbeitungsphase (P2), umfassend eine Identifizierung (S9) des chirurgischen Instruments (DMᵢ), die insbesondere aus Folgenden besteht: Vergleichen (S9_1) des Gewichtsdatums (D_PDSᵢ) und des visuellen Datums (D_PICᵢ) mit einer Datenbank (DB), die eine Verbindung zwischen mindestens einem chirurgischen Instrument (DMᵢ) und den Informationen hinsichtlich des Gewichts und der visuellen Eigenschaft des mindestens einen chirurgischen Instruments festsetzt, und Bestimmen (S9_2), in Funktion des Vergleichs, mindestens eines Identifizierungsdatums (DIᵢ), das eine Information hinsichtlich der Identität des chirurgischen Instruments (DMᵢ) enthält,
**dadurch gekennzeichnet, dass** die Datenbank (DB) für jedes chirurgische Instrument (DMᵢ) ein Lokalisierungsdatum (DLᵢ) umfasst, das eine Information hinsichtlich eines vorbestimmten Standorts (Pᵢ) des chirurgischen Instruments (DMᵢ) in einem chirurgischen Kit (KT) enthält, und dadurch, dass es eine Neuzusammensetzungsphase (P3) des Kits (KT) umfasst, aufweisend:
- einen Schritt des Lokalisierens (10), im Laufe dessen der Standort (Pᵢ) des chirurgischen Instruments (DMᵢ) in dem chirurgischen Kit (KT) in Funktion des Lokalisierungsdatums (DLᵢ) bestimmt wird, und
- einen Schritt des Anzeigens (S11), insbesondere bestehend aus dem Anzeigen auf einem digitalen Bildschirm (80), einer digitalen Kartographie des chirurgischen Kits (KT) sowie der chirurgischen Instrumente (DMᵢ), die gemäß ihrem Standort (Pᵢ) assoziiert sind.

2. Verfahren nach Anspruch 1, wobei die Stützplattform (10), auf die das chirurgische Instrument (DMᵢ) gestellt ist, zerstreuend ist, und wobei die Erfassungsphase (P1) eine Hintergrundbeleuchtung (S2) des chirurgischen Instruments (DMᵢ) aufweist, indem ein Hintergrundbeleuchtungs-Licht (LUX) über die Plattform (10) und in Richtung der Digitalkamera (20) emittiert wird.

3. Verfahren nach Anspruch 2, wobei das Hintergrundbeleuchtungs-Licht (LUX) eine Wellenlänge aufweist, die im Bereich zwischen 400 und 500 Nanometer liegt, vorzugsweise im Wesentlichen gleich 470 Nanometer ist.

4. Verfahren nach Anspruch 2 oder 3, wobei die Erfassungsphase (P1) eine Polarisierung (S3) des Hintergrundbeleuchtungs-Lichts (LUX) durch einen einseitig ausgerichteten Diffusorfilm (11) aufweist, der die Plattform (10) bedeckt, um die Diffusion des Hintergrundbeleuchtungs-Lichts, das über die Plattform (10) strömt, zu homogenisieren.

5. Verfahren nach einem der vorhergehenden Ansprüche, wobei die Erfassungsphase (P1) eine Messung (S7) der Impedanz des chirurgischen Instruments (DMᵢ) aufweist, und wobei die Identifizierung (S9) die Impedanz in Betracht zieht, um das Identifizierungsdatum (DIᵢ) zu bestimmen, wobei die Datenbank (DB) für jedes chirurgische Instrument (DMᵢ) eine Information hinsichtlich eines Werts der vorbestimmten Impedanz aufweist.

6. Verfahren nach einem der vorhergehenden Ansprüche, wobei die Verarbeitungsphase (P2) einen vorherigen Schritt des Aggregierens (S8) des Gewichtsdatums (D_PDSᵢ) und des Bilddatums (D_PICᵢ) umfasst.

7. Verfahren nach einem der vorhergehenden Ansprüche, aufweisend einen vorherigen Schritt des Erlernens (S0), im Laufe dessen die Datenbank (DB) konstruiert wird, indem für jedes bestimmte chirurgische Instrument (DMᵢ) die Informationen hinsichtlich des Gewichts und mindestens einer visuellen Eigenschaft des chirurgischen Instruments assoziiert werden.

8. Verfahren nach einem der vorhergehenden Ansprüche, **dadurch gekennzeichnet, dass** die Datenbank (DB) eine zentralisierte Datenbank ist, die mit einem entfernten Server (S) verbunden ist, und wobei der Schritt des Identifizierens (S9) die Fernabfrage (S9_0) dieser Datenbank (DB) aufweist.

9. Informatikvorrichtung zur Nachverfolgbarkeit (100) der chirurgischen Instrumente (DMᵢ, wobei i eine ganze positive Zahl ist) auf einem Krankenhausgelände, wobei die Vorrichtung (100) Folgendes umfasst:
- ein Erfassungsmodul (M1), aufweisend:
eine Stützplattform (10), die ausgelegt ist, um ein chirurgisches Instrument (DMᵢ, wobei i eine ganze positive Zahl ist) zu empfangen,
eine Digitalkamera (20), die über der Stützplattform (10) positioniert ist, die konfiguriert ist, um mindestens ein Digitalbild (I) zu erstellen, das mindestens einen Abschnitt des chirurgischen Instruments (DMᵢ) aufweist,
eine elektronische Schaltung zur Bildverarbeitung (30), die konfiguriert ist, um in dem mindestens einen Digitalbild (I) mindestens eine visuelle Eigenschaft des chirurgischen Instruments (DMᵢ) nachzuweisen, und um mindestens ein visuelles Datum (P_PICᵢ) zu bestimmen, das mit der visuellen Eigenschaft assoziiert ist,
eine digitale Waage (40), die konfiguriert ist, um das Gewicht des chirurgischen Instruments (DMᵢ) zu messen und um ein Gewichtsdatum (D_PDSᵢ) zu bestimmen, das die Information hinsichtlich des gemessenen Gewichts enthält;
- ein Behandlungsmodul (M2), aufweisend:
eine elektronische Schaltung zur Identifizierung (60), die einerseits konfiguriert ist, um das Gewichtsdatum (D_PDSᵢ) und das visuelle Datum (D_PICᵢ) mit einer vorbestimmten Datenbank (DB), die eine Verbindung zwischen mindestens einem chirurgischen Instrument (DMᵢ) und den Informationen hinsichtlich des Gewichts und der visuellen Eigenschaft des mindestens einen chirurgischen Instruments festsetzt, zu vergleichen, und andererseits, um in Funktion des Vergleichs mindestens ein Identifizierungsdatum (DIᵢ) zu bestimmen, das eine Information hinsichtlich der Bezeichnung des chirurgischen Instruments (DMᵢ) enthält,
**dadurch gekennzeichnet, dass** die Datenbank (DB) für jedes chirurgische Instrument (DMᵢ) ein Lokalisierungsdatum (DLᵢ) umfasst, das eine Information hinsichtlich eines vorbestimmten Standorts (Pᵢ) des chirurgischen Instruments (DMᵢ) in einem chirurgischen Kit (KT) enthält, und dadurch, dass es eine elektronische Schaltung zur Nachverfolgbarkeit (90) umfasst, die konfiguriert ist, um den Standort (Pᵢ) des chirurgischen Instruments (DMᵢ) in dem chirurgischen Kit (KT) in Funktion des Lokalisierungsdatums (DLᵢ) und der Anzeigemittel (80), wie z. B. einem Digitalbildschirm, zu bestimmen, die konfiguriert sind, um eine digitale Kartographie des chirurgischen Kits (KT) anzuzeigen, ebenso wie die chirurgischen Instrumente (DMᵢ), die gemäß ihrem Standort (Pᵢ) assoziiert sind.

10. Vorrichtung zur Nachverfolgbarkeit (100) nach Anspruch 9, umfassend Informatikmittel, die konfiguriert sind, um Schritte des Verfahrens nach einem der Ansprüche 2 bis 8 durchzuführen.

## Claims

1. A method for tracing surgical instruments (DMᵢ, i being a positive integer) in a hospital environment, said method being implemented by computer processing means comprising:
- an acquisition phase (P1) comprising:
positioning (S1) a surgical instrument (DMᵢ) on a support platform (10) ;
capturing (S4), by means of a digital camera (20) positioned above the support platform (10), at least one digital image (I) comprising at least one portion of the surgical instrument (DMᵢ); and
image processing (S5) for detecting in said at least one digital image (I) at least one visual characteristic of said surgical instrument (DMᵢ) and for determining at least one piece of visual data (P_PlCᵢ) associated with said visual characteristic;
measuring (S6) the weight of the surgical instrument (DMi), by means of a digital scale (40), in order to obtain information about the weight (D_PDSᵢ) containing information relating to the measured weight, and
- a processing phase (P2) comprising an identification (S9) of said surgical instrument (DMᵢ) consisting in particular of: comparing (S9_1) the weight information (D_PDSᵢ) and the visual information (D_PICᵢ) with a database (DB) establishing a link between a least one surgical instrument (DMᵢ) and information relating to the weight and to the visual characteristic of said at least one surgical instrument, and determining (S9_2), as a function of said comparison, at least one piece of identification information (DIᵢ) containing information relating to the identity of said surgical instrument (DMᵢ),
wherein the database (DB) comprises for each surgical instrument (DMᵢ), a localization data (DLᵢ) containing information relating to a predetermined position (Pᵢ) of said surgical instrument (DMi) in a surgical kit (KT), and
wherein said method comprising a reassembly phase (P3) of said kit (KT) comprising :
- a localization stage (10) during which the position (Pi) of said surgical instrument (DMi) in said surgical kit (KT) is determined according to said localization data (DLᵢ), and
- a display stage (S11) consisting in particular of displaying on a digital touch screen (80) a digital inventory of the surgical kit (KT) as well as associated surgical instruments (DMᵢ) according to their position (Pᵢ).

2. The method according to claim 1, wherein the support platform (10) on which said surgical instrument (DMi) is placed is diffusing, and wherein the acquisition phase (P1) comprises backlighting (S2) the surgical instrument (DMi) by emitting a backlight (LUX) through said platform (10) and in the direction of said digital camera (20).

3. The method according to claim 2, wherein the backlight (LUX) has a wavelength between in the order of 400 and 500 nanometers, preferably substantially 470 nanometers.

4. The method according to claim 2 or 3, wherein the acquisition phase (P1) comprises a polarization (S3) of the backlight (LUX) by a unidirectional diffusing film (11) covering said platform (10) for homogenising the diffusion of the backlighting passing through said platform (10).

5. The method according to any of the preceding claims, wherein the acquisition phase (P1) comprises measuring (S7) the impedance of said surgical instrument (DMᵢ), and wherein the identification (S9) takes into account said impedance for determining the identification (Dlᵢ), said database (DB) comprising for each surgical instrument (DMi) information relating to a predetermined impedance value.

6. The method according to any of the preceding claims, wherein the processing phase (P2) comprises a previous gathering stage (S8) of the weight data (D_PDSᵢ) and the pictorial date (D_PlCᵢ).

7. The method according to any of the preceding claims, comprising an earlier teaching stage (S0) during which the database (DB) is created by associating for each surgical instrument (DMi) information relating to the weight and at least one visual characteristic of said surgical instrument.

8. The method according to any of the preceding claims, wherein the database (DB) is a centralized database connected to a remote server (S), and wherein the identification stage (S9) comprises querying (S9_0) remotely said database (DB).

9. A computer device for tracing (100) surgical instruments (DMᵢ, i being a positive integer) in a hospital environment, said device (100) comprising :
- an acquisition module (M1) comprising :
a support platform (10) designed to receive a surgical instrument (DMᵢ, i being a positive integer),
a digital camera (20) positioned above the support platform (10), configured to capture at least one digital image (I) comprising at least one portion of the surgical instrument (DMᵢ),
an electronic circuit for processing images (30) configured to detect in said at least one digital image (I) at least one visual characteristic of said surgical instrument (DMᵢ) and to determine at least one piece of visual data (P_PICᵢ) associated with said visual characteristic,
a digital scale (40) designed to measure the weight of the surgical instrument (DMᵢ) and determine weight data (D_PDSᵢ) containing information relating to the measured weight;
- a processing module (M2) comprising an electronic identification circuit (60) configured on the one hand for comparing the weight data (D_PDSᵢ) and the visual data (D_PICᵢ) with a predetermined database (DB) establishing a link between at least one surgical instrument (DMᵢ) and information relating to the weight and said visual characteristic of said at least one surgical instrument, and on the other hand for determining according to said comparison at least one piece of identification data (DIᵢ) containing information relating to the designation of said surgical instrument (DMᵢ) ;
wherein the database (DB) comprises for each surgical instrument (DMᵢ), a localization data (DLᵢ) containing information relating to a predetermined position (Pᵢ) of said surgical instrument (DMᵢ) in a surgical kit (KT), and
wherein said device (100) comprising :
- a tracing electronic circuit (90) configured for determining the position (Pi) of said surgical instrument (DMᵢ) in said surgical kit (KT) according to said localization data (DLᵢ), and
- display means (80) such as a digital screen configured to display a digital inventory of the surgical kit (KT) as well as associated surgical instruments (DMᵢ) according to their position (Pᵢ).

10. The device according to claim 9, comprising means configured for executing the steps of the method as claimed in any of claims 2 to 8.
